# EUROPÄISCHE PATENTSCHRIFT

(11) **EP 0 892 925 B1**
(45) Veröffentlichungstag und Bekanntmachung des Hinweises auf die Patenterteilung: **12.09.2001**
(21) Anmeldenummer: 97920623.2
(22) Anmeldetag: 07.04.1997
(51) Int. Cl.: G01N 33/36

(54) **VERFAHREN UND VORRICHTUNG ZUM NACHWEIS UNTEXTURIERTER GARNABSCHNITTE IN TEXTURIERTEN FILAMENTGARNEN**
METHOD AND DEVICE FOR DETECTION OF UNTEXTURED YARN SECTIONS IN TEXTURED FILAMENT YARNS
PROCEDE ET DISPOSITIF POUR DETECTER DES SECTIONS DE FIL NON TEXTUREES DANS DES FILS CONTINUS

(30) Priorität: 09.04.1996 DE 19614027; 09.04.1996 DE 19614026
(43) Veröffentlichungstag der Anmeldung: 27.01.1999
(73) Patentinhaber: DEUTSCHE INSTITUTE FÜR TEXTIL- UND FASERFORSCHUNG STUTTGART Stiftung des öffentlichen Rechts, 73779 Denkendorf (DE)
(72) Erfinder: WEINSDÖRFER, Helmut, D-72124 Pliezhausen (DE); CUI, Beiheng, D-70569 Stuttgart (DE)
(74) Vertreter: Götz, Georg, Dipl.-Ing.
(86) Internationale Anmeldenummer: EP9701729
(87) Internationale Veröffentlichungsnummer: WO9738306

(56) Entgegenhaltungen:
- EP-A- 0 495 446
- EP-A- 0 531 753
- EP-A- 0 555 639
- DE-A- 4 119 780
- US-A- 4 295 360

## Beschreibung

Die Erfindung betrifft ein Verfahren zum Nachweis von untexturierten beziehungsweise fehlerhaft texturierten Garnabschnitten in texturierten Filamentgarnen während des Texturierprozesses sowie eine Vorrichtung zur Durchführung dieses Verfahrens.

Das Texturieren von Filamentgarnen, insbesondere Multifilamentgarnen, ist bekannt. Das Texturieren dient dazu, aus einem kunststoffartigen, flachen und glatten Multifilamentgarn ein gekräuseltes und strukturiertes Garn herzustellen, das aufgrund seiner voluminösen und bauschigen Struktur textilen Charakter aufweist. Dazu wird das Multifilamentgarn im allgemeinen von einer Spule abgewickelt, durch ein erstes Lieferwerk geführt, anschließend in einem Heizer erhitzt, auf einer Kühlschiene abgekühlt, durch einen Drallgeber und ein dahinter angeordnetes zweites Lieferwerk, ein sogenanntes Abzugslieferwerk geführt, um anschließend auf einer Garnspule aufgewickelt zu werden. Der Drallgeber dient dazu, das Multifilamentgarn in einem Arbeitsgang vorübergehend hochzudrehen (eine vorübergehende Drehung wird auch als Falschdraht bezeichnet) und ermöglicht es durch den sich gleichzeitig ausbildenden Drehungsrückstau, den tordierten Zustand im Bereich vor dem Drallgeber durch Erhitzen und Abkühlen thermisch zu fixieren. Hinter dem Drallgeber ist die Drehung wieder herausgenommen und das Filamentgarn aufgedreht. Aufgrund der im tordierten Zustand erfolgten thermischen Fixierbehandlung weist das Garn die gewünschte gekräuselte Struktur auf.

Die Drallerteilung erfolgt vorwiegend mit einem dreiachsigen Scheibenfriktionsaggregat oder mittels sogenannter gekreuzter Riemchen. Die Drallerteilung mittels Friktion ermöglicht sehr hohe Rotations- und damit auch hohe Produktionsgeschwindigkeiten. Sind jedoch die Reibungsverhältnisse zwischen Drallgeber und Garn nicht konstant, dann führt dies zu Störungen des Prozesses, die auch als Instabilitäten bezeichnet werden und damit zu Qualitätsverlusten im Garn führen. Derartige Fehler können aus Störungen in der Spinnerei, aus ungleichmäßigem Auftrag oder ungleichmäßiger Verteilung der Spinnpräparation auf der Fadenoberfläche, aus Temperaturschwankungen beim Texturieren oder auch aus Verschmutzungen von Heiz- und/oder Kühlschienen resultieren. Die Störungen können ein sogenanntes Ballonieren des Gams bewirken, was insbesondere bei hohen Rotationsgeschwindigkeiten auftritt. Ein Ballonieren des Gams führt zu einem unkontrollierten Fadenlauf, zu Fadenspannungsschwankungen und zu Qualitätsverlusten. Insbesondere bei instabilen Prozessen kann der Faden über die Scheibenoberfläche des Friktionsaggregats springen. Dieser sogenannte Drallschlupp führt zu einem Drehungsdefizit innerhalb der Drallzone. Vor dem Drallgeber hochgedrehtes Garn kann also, kurzzeitig und/oder abschnittsweise ohne Auflösung der Drehung, das Aggregat passieren. Dies führt zu kurzen, untexturierten Garnabschnitten (kurzzeitiger Drallschlupf), sogenannten "tight spots" und langen, untexturierten Garnabschnitten ("Surging", langzeitiger Drallschlupf).

Die Qualität des texturierten Garns wird im allgemeinen durch stichprobenartige Prüfung des fertig texturierten Garns kontrolliert. Dabei kann nur ein sehr kleiner Bruchteil der gesamten Produktionsmenge geprüft werden. In den letzten Jahren wurden sogenannte On-Line-Produktionskontrollen beim Texturieren eingeführt, so daß weitgehend die gesamte Produktionsmenge auf ihre Qualität hin kontrolliert werden kann. Dabei werden jedoch die Garne nicht lückenlos meßtechnisch erfaßt, weil die verwendeten Meßsensoren und die Abtastraten niedrigfrequent arbeiten, und so unerwünschte kurze Störstellen ("tight spots") häufig nicht erkannt werden. Die On-Line-Kontrolle im Texturierprozeß wird im allgemeinen mittels Fadenzugkraftmessung durchgeführt. Bekannt sind Kontrollsysteme, die niedrigfrequente Fadenzugkraftschwenkungen beziehungsweise -spitzen erfassen können, das heißt Fadenzugkraftschwankungen, die auf längeren Fehlstellen im Garn beruhen. Die Erfassung von kurzen Störungen und Fehlstellen ist mit diesen Systemen nicht möglich.

Zur Ermittlung kurzperiodischer Fadenzugkraftschwankungen ist ein Fadenzugkraftsensor für eine Textilmaschine bekannt geworden (EP 0 531 753 A1). Mit diesem soll bei hohen Fadengeschwindigkeiten auch eine Messung relativ hochfrequenter Fadenzugkraftschwankungen zum Beispiel bis zu 50 Hertz möglich sein. Damit können auch durch die Fadenverlegung bewirkte, relativ hochfrequente Fadenzugkraftschwankungen erfaßt werden. So soll allgemein für den Fadenspannungssensor eine erheblich größere Anwendungsbreite erzielt werden. Als Beispiele dafür werden der Ausgleich nichtperiodischer Fadenspannungsschwankungen über Fadenbremsmittel, Erzeugung von Störsignalen bei periodisch auftretenden Abweichungen der vorgegebenen Fadenzugkraftcharakteristik sowie Erkennen der progressiven Zunahme der Fadenzugkraft beim Aufspulen des letzten Drittels eines Kopses an der Spulstelle mit entsprechender Drosselung der Spulgeschwindigkeit zur Vermeidung von Fadenbrüchen offenbart.

In der Offenlegungsschrift DE 41 19 780 wird zur Qualitätsüberwachung in einer Falschzwirnkräuselmaschine vorgeschlagen, laufend die Fadenzugkraft zwischen dem Falschdraller und dem Ausgangslieferwerk der Texturierzone zu messen. Aus den Meßwerten wird ständig ein Mittelwert gebildet und seine Lage und/oder die Relativlage des laufenden Meßwertes zu dem Mittelwert ausgewertet. Um eine Aussage über die Art der die Qualität beeinflußenden Fehler machen zu können, wird ferner vorgeschlagen, mehrere durch die Länge der Auswertzeit unterscheidbare Mittelwerte zu bilden und zur Erzeugung eines Qualitätssignals die Relativlage des Meßwertes zu einem oder mehreren der Mittelwerte und/oder die Relativlage der Mittelwerte untereinander zu bestimmen. Allerdings können mit dieser Methode plötzliche, kurzzeitige Prozeßunregelmäßigkeiten wie Spannungspitzen, die zum Beispiel bei Ablaufhindernissen auf der Spule für den darauf ablaufenden Faden auftreten und anschließend wieder völlig verschwinden, mit der Methode der Mittelwertbildung nicht gezielt und eigenständig erfaßt sowie ausgewertet werden.

Auch nach EP 0 406 736 B1 wird zur Überwachung der Fadenzugkraft eines laufenden Fadens die ununterbrochene Bestimmung des Mittelwerts vorgeschlagen. Ferner ist ständig ein Differenzwert aus dem Mittelwert und dem aktuellen Fadenzugkraftwert zu bilden. Immer dann, wenn der Mittelwert und/oder der Differenzwert vorgegebene Toleranzbereiche für vorbestimmte Zeiten verlassen, werden entsprechende Alarmsignale erzeugt. Abhängig von deren Auftreten während vorbestimmter Zeiten werden Qualitätsangaben für den faden abgeleitet. Allerdings werden dabei Zeitverzögerungsglieder mit Verzögerungszeitkonstanten von ungefähr 10 Millisekunden eingesetzt, um Ausgangssignale auszufiltern, die auf kurzzeitige und als irrelevant eingestufte Störungen des Garn-Texturierprozesses zurückgehen.

Der vorliegenden Erfindung liegt also das technische Problem zugrunde, ein verbessertes Verfahren und eine verbesserte Vorrichtung zum Nachweis von unerwünschten, untexturierten beziehungsweise fehlerhaft texturierten Garnabschnitten, insbesondere kurzer Längen in insbesondere nach dem Friktionsfalschdraht-Verfahren hergestellten texturierten Filamentgamen bereitzustellen, das während des Texturierens durchgeführt wird.

Die Lösung des technischen Problems liegt in der Bereitstellung eines Verfahrens gemäß Anspruch 1 zum Nachweis von untexturierten beziehungsweise fehlerhaft texturierten Garnabschnitten in texturierten Filamentgarnen während des Texturierprozesses. wobei hochfrequente Fadenzugkraftsignale, gemessen und ausgewertet werden. Die gemessenen Fadenzugkraftsignale erlauben Aussagen über den Grad der Garnkräuselung und deren Gleichmäßigkeit. Störstellen im Garn, insbesondere auch kurze untexturierte Störstellen, werden durch das während des Texturierprozesses stattfindende Nachweisverfahren als entsprechend kurzzeitige Signale bestimmt, die erfindungsgemäß als Nachweis für derartige Störstellen gemessen und ausgewertet werden. Im Zusammenhang mit der vorliegenden Erfindung wird unter einer kurzen Störstelle ("tight spot") eine Störstelle der Länge von etwa maximal 50 mm, vorzugsweise 1 bis 50 mm, verstanden. Im Zusammenhang mit der vorliegenden Erfindung werden unter hochfrequenten Fadenzugkraftsignalen Fadenzugkraftspitzen oder -schwankungen verstanden, die bei gegebener Gamlaufgeschwindigkeit eine Frequenz von 0,2 kHz, bevorzugt von 1 bis 6 kHz, aber auch höhere Frequenzen bis 10 kHz aufweisen.

Das erfindungsgemäße Verfahren sieht vor, die hochfrequenten Fadenzugkraftsignale zu messen, aufzubereiten und auszuwerten und so Aussagen über den Grad der Garnkräuselung und deren Gleichmäßigkeit zu erlauben.

Da das erfindungsgemäße Verfahren hochfrequente Fadenzugkraftsignale nachweist, können insbesondere auch kurzzeitige Störungen registriert werden, die durch sogenannte "tight spots" entstehen, so daß bereits frühzeitig, das heißt, bevor die Garnqualität spürbar nachläßt, festgestellt werden kann, wann ein Texturierprozeß instabil und damit fehlerbelastet wird.

Die Erfindung sieht vorzugsweise vor, daß die Fadenzugkraft des texturierten Garns nach Durchlaufen des Drallgebers gemessen wird, das heißt in einem Zustand, in dem das Garn grundsätzlich wieder aufgedreht ist. Die Durchführung des erfindungsgemäßen Verfahrens mit aufgedrehtem Garn, das heißt nach Durchlaufen des Drallgebers, ist besonders vorteilhaft, da die Drallerteilung und damit der Texturierprozeß durch die für die Fadenzugkraftmessung erforderliche Umlenkung des Garns am Meßgeber nicht beeinträchtigt wird.

In bevorzugter Ausführungsform wird das erfindungsgemaße Nachweisverfahren während des Friktionsfalschdraht-Verfahrens eingesetzt. Der Drallgeber ist in diesem Fall ein Scheibenfriktionsaggregat.

Das erfindungsgemäß vorgesehene Erfassen kurzzeitiger, während des Texturierprozesses auftretender Störungen über hochfrequente Fadenzugkraftsignale, wird mittels eines Meßsensors ermöglicht, der in der Lage ist, hochfrequente Fadenzugkraftschwankungen beziehungsweise -spitzen aufzunehmen. Die gemessenen hochfrequenten Fadenzugkraftsignale werden aufbereitet und ausgewertet und geben so erfindungsgemäß Auskunft über den Grad der Garnkräuselung und deren Gleichmäßigkeit. Die Auswertung der gemessenen Fadenzugkraftsignale wird beispielsweise durchgeführt, indem ein Schwellenwert vorgegeben wird und die pro Zeiteinheit auftretenden Überschreitungen des Schwellenwerts erfaßt werden. Die Anzahl der Überschreitungen ist ein Maß für Unregelmäßigkeiten und Störungen. Die Höhe des Mittelwerts ist ein relatives Maß für die Intensität der Gamkräuselung.

Das erfindungsgemäße Verfahren ermöglicht also in vorteilhafter Weise den Nachweis insbesondere kurzer Störstellen, die durch Messung hochfrequenter Fadenzugkraftsignale nachgewiesen werden.

Die Erfindung betrifft auch eine im unabhängigen Anspruch 17 angegebene Vorrichtung zum Nachweis von untexturierten beziehungsweise fehlerhaft texturierten Garnabschnitten in texturierten Filamentgarnen, insbesondere zur Durchführung des vorgenannten Verfahrens, wobei die Vorrichtung einen Meßsensor, eine Vorrichtung zur Signalaufbereitung und -verarbeitung sowie zur Signalanalyse, Datenverarbeitung und gegebenenfalls zur Datenausgabe aufweist, und wobei der Meßsensor in der Lage ist, kurzzeitige Fadenzugkraftsignale zu detektieren und einer Datenauswertung zuzuführen. Die Auswertung wird durch die erfindungsgemäße Vorrichtung durchgeführt, indem deren Signalverarbeitungsvorrichtung heben optionalen Mittel zur wählbaren Glättung, beziehungsweise Filterung Mittelwertbildung, Bildung von Streuungsquadraten, und/oder Differentialbildung Vorrichtungen zur wählbaren Schwellenwertvorgabe und/oder Zählung der Schwellenwertüberschreitungen im aufbereiteten Signal aufweist, wobei letztere Zählschaltung die Anzahl der in einer bestimmten Zeit den Schwellenwert überschreitenden hochfrequenten Signal-Ausschläge erfaßt. Die Anzahl der Ausschläge pro Zeiteinheit ist als Maß für die Ungleichmäßigkeit der Garnkräuselung zu werten. Die erfindungsgemäße Vorrichtung ermöglicht also den Nachweis hochfrequenter Fadenzugkraftsignale, und die anschließende Auswertung unter Berücksichtigung des erfindungsgemäß erkannten Zusammenhanges zwischen dem Auftreten kurzer Störstellen und von hochfrequenten Fadenzugkraftsignalen. Die erfindungsgemäß einzusetzenden Meßsensoren, sind in der Lage, Fadenzugkraftsignale im Frequenzbereich von 0,2 kHz bis 6 kHz oder auch in höheren Frequenzbereichen aufzunehmen.

Die gemessenen hochfrequenten Faderzugkraftsignale können dann beispielsweise weiter mittels FFT-Analyse (Fast Fourier-Transformation) des Fadenzugkraftsignals ausgewertet werden, so daß auch der Nachweis von kleinsten, periodischen Schwankungen möglich ist, die beispielsweise im normalen zeitlichen Fadenzugkraftverlauf nicht sichtbar sind. Die Verwendung hoher Abtastraten, beispielsweise von 50 kHz oder 250 kHz, vermeidet das Auftreten von Aliasing-Effekten bei der FFT-Analyse.

Da das erfindungsgemäße Verfahren das Erfassen einer großen Anzahl von Meßsignalen umfaßt, ist erfindungsgemäß auch vorgesehen, die analogen Meßsignale vorzugsweise bereits an der Meßstelle zur Datenverdichtung aufzubereiten und teilweise auszuwerten, und dann die digitalen Ergebnisse einem Zentralcomputer zur Speicherung, rechnergestützten Gesamtauswertung und Dokumentation zu übermitteln. Die Auswertung kann durch Aufnahme beziehungsweise Bestimmung von Amplitudenspektren, Autokorrelationsfunktionen, zeitlichen Fadenzugkraftverläufen, Mittelwerten, Standardabweichungen, Variationskoeffizienten, Streuungsquadraten, FFT-Analysen und/oder Amplitudenhistogrammen des Fadensignals insbesondere Fadenzugkraftsignals durchgeführt werden.

Alternativ oder zusätzlich liegt die Lösung des obigen Problems in einem im unabhängigen Anspruch 9 angegebenen Verfahren zum Nachweis von untexturierten beziehungsweise fehlerhaft texturierten Gamabschnitten in texturierten Filamentgarnen während des Texturierprozesses, wobei die Garndicke optisch oder laseroptisch bestimmt und anschließend ausgewertet wird. Die Überprüfung der Garndicke gibt in vorteilhafter Weise Aufschluß über den Texturierprozeß und die Qualität des erhaltenen Garnes, da optisch oder laseroptisch erkennbare vergleichsweise dünne Garnstellen auf untexturierte, beziehungsweise fehlerhaft texturierte, nicht aufgedrehte Gamabschnitte hinweisen. Erfindungsgemäß deuten insbesondere Gamdickenabweichungen von mehr als 20 % auf kurze, untexturierte Garnstellen hin. Insbesondere kurze Störstellen konnten mit den bisher bekannten Verfahren nicht nachgewiesen werden. was nun mit Hilfe der vorliegenden Erfindung in vorteilhafter Weise möglich ist. In vorteilhafter Weise können also kurze Instabilitäten, beispielsweise das Rutschen des Fadens auf einer Scheibe des Friktionsaggregates, die zu "tight spots" führen, nachgewiesen werden.

Die Erfindung sie auch ein im unabhängigen Anspruch 10 angegebenen Verfahren zum Nachweis von untexturierten beziehungsweise fehlerhaft texturierten Garnabschnitten in texturierten Filamentgarnen während des Texturierprozesses vor, wobei alternativ oder zusätzlich zur Garndickenbestimmung die Garnposition und/oder Garnpositionsveränderungen, insbesondere Garnquerlaufschwankungen optisch und/oder laseroptisch bestimmt und ausgewertet werden. Die Verwendung optischer oder laseroptischer Vorrichtungen ermöglicht es, auch in dieser Ausführungsform der Erfindung kurzzeitige Instabilitäten, die zu unerwünschten, kurzen Störungen führen, nachzuweisen. Die vorliegende Erfindung beruht also unter anderem darauf, erkannt zu haben, daß die Garnposition beziehungsweise die Veränderung der Garnposition beim Lauf über eine Scheibenoberfläche des vorzugsweise als Friktionsaggregates ausgeführten Drallgebers den Nachweis von kurzen, untexturierten beziehungsweise fehlerhaft texturierten Garnabschnitten ermöglicht. Erfindungsgemäß ist besonders bevorzugt vorgesehen, das Garnlaufverhalten auf der Ausgangsscheibe, also der letzten Friktionsscheibe -meist ist diese eine Fadenführungsscheibe- des Friktionsaggregates zu bestimmen. Bei einem stabilen Texturierprozeß, das heißt einem Texturierprozeß ohne Bildung von Störstellen, läuft der Faden an der Ausgangsscheibe auf einer stärker gekrümmten Kurve, die anzeigt, daß auf den Faden eine Reibkraft einwirkt. Die Krümmung des Garnlaufs verkleinert sich bei einem leicht instabilen Prozeß, wobei bei einem sehr instabilen Prozeß der Faden fast gerade über die letzte Scheibe läuft, was anzeigt, daß die einwirkende Reibkraft gegen 0 geht. Bei einem instabilen Prozeß treten insbesondere Veränderungen des Garnlaufwegs auf der Ausgangsscheibe auf, die wie eine Welle erscheinen. Daraus ist erkennbar, daß beim instabilen Prozeß der Faden über die Scheibenoberfläche springt. Dieser Drallschlupf führt zu einem Drehungsdefizit innerhalb der Drallzone. Tritt diese Welle auf der Ausgangsscheibe auf, kann hochgedrehtes Garn kurzzeitig, ohne Auflösung der Drehung, das Aggregat passieren und "tight spots" bilden.

Die optische oder laseroptische Überprüfung des -Garnlaufverhaltens, insbesondere auf der Ausgangsscheibe, ermöglicht also den Nachweis von kurzen Störstellen im texturierten Garn. Der Nachweis kurzer untexturierter Garnabschnitte durch Bestimmung und Auswertung der Garnposition und des Garnlaufverhaltens kann sowohl alleine als auch in Kombination mit der bereits beschriebenen Garndikke-Nachweismethode zur Bestimmung kurzer untexturierter Garnabschnitte verwendet werden. Erfindungsgemäß werden sowohl für die Bestimmung der Garndicke als auch für die Bestimmung der Garnposition und/oder des Gamlaufverhaltens optische und/oder taseroptische Vomchtungen verwendet, die in der Lage sind, kurze Störstellen im Garn nachzuweisen, also hohe Abtastraten, vorzugsweise > 10 kHz. aufweisen. Erfindungsgemäß sind insbesondere Abtastraten zu wählen, die so hoch sind, daß bei der gewählten Produktionsgeschwindigkeit die Gamdicke in 1 mm-Abständen abgetastet wird und Gampositionsschwankungen von 6 kHz und mehr erfaßt werden können. Je höher die Abtastraten und die Auflösung der verwendeten optischen und/oder laseroptischen Vorrichtungen sind. desto kürzere Störstellen können erfaßt und/oder bei desto höheren Produktionsgeschwindigkeiten kann das Verfahren angewandt werden. Die Garndicke kann mit Hilfe einer Photozelle, die Veränderung der Garnposition mittels einer Photozellenleiste (CCD-Zeile) bestimmt werden.

Gemäß einer weiteren bevorzugten Ausführungsform der Erfindung ist vorgesehen, sowohl die Bestimmung der Garndicke, als auch die Bestimmung der Gamposition und/oder des Garnlaufverhaltens während der Drallerteilung im Bereich der Friktionsscheiben des Scheibenfriktionsaggregates durchzuführen, insbesondere die Bestimmung auf oder an der letzten Arbeitsscheibe oder der letzten Fadenführungsscheibe, das heißt der Ausgangsscheibe, durchzuführen. Insbesondere das Garnlaufverhaiten weist, je nach Instabilität des Texturierprozesses, deutliche Unterschiede an beziehungsweise auf der Ausgangsscheibe auf, so daß die optischen oder laseroptischen Messungen vorzugsweise dort durchzuführen sind. Die Erfassung der Gamdicke kann jedoch auch auf der gesamten Strecke von der Ausgangsscheibe des Drallgebers bis zur Aufwindung des texturierten Garns auf der Spule erfolgen.

Selbstverständlich ist es auch möglich, mittels der beschriebenen, erfindungsgemäßen Verfahren, lange untexturierte Garnabschnitte (sogenannte "Surging-Stellen") nachzuweisen.

Weitere vorteilhafte Ausgestaltungen der Erfindung sind den Unteransprüchen zu entnehmen.

Weitere Einzelheiten, Merkmale und Vorteile auf der Basis der Erfindung ergeben sich aus der folgenden Beschreibung von Ausführungsbeispielen anhand der Zeichnungen. Diese zeigen in:
- Fig. 1: schematisch eine Anordnung zum Texturieren von Multifilamentgarnen;
- Fig. 2A und 2B: schematisch das Prinzip der Fadenzugkraftbestimmung;
- Fig. 3: den Aufbau einer erfindungsgemäßen Vorrichtung in Form eines Blockdiagrammes;
- Fig. 4A und 4B: den zeitlichen Fadenzugkraftverlauf vor und nach Einsatz eines Tiefpaßfilters und
- Fig. 5A bis 5G: Amplituden-Spektren der Fadenzugkraft F2 nach dem Drallgeber in stabilen und instabilen Prozessen.
- Fig. 6: ein Friktionsaggregat und
- Fig. 7: schematisch die Fadenlaufgeometrie auf der Ausgangsscheibe eines Friktionsaggregates.

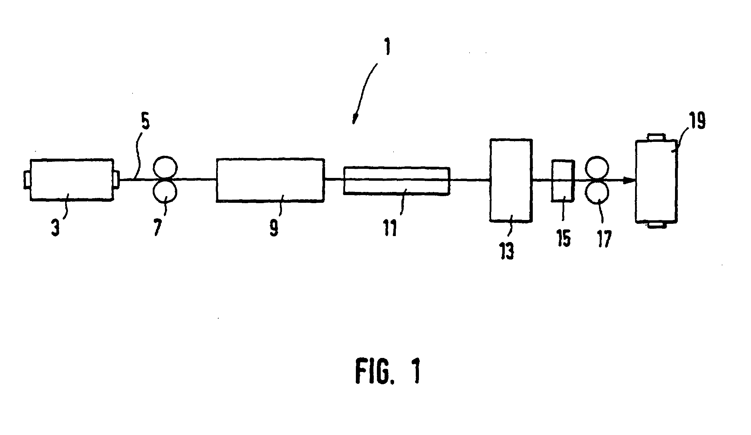

Die Figur 1 zeigt schematisch eine Vorrichtung 1 zum Texturieren von Filamentgarnen mittels eines Friktionsfalschdraht-Verfahrens. Die Vorrichtung 1 weist eine Spule 3 auf, von der das Multifilamentgarn 5 abgespult wird und durch ein Lieferwerk 7 in einen Heizer 9 geführt wird. Das Multifilamentgarn 5 wird aus dem Heizer 9 über eine Kühlschiene 11 und durch einen als Scheibenfriktionsaggregat ausgeführten Drallgeber 13 in ein Abzugslieferwerk 17 und von dort auf die Garnspule 19 geführt. Zwischen Drallgeber 13 und Abzugslieferwerk 17 befindet sich eine Vorrichtung 15 zum Nachweis von unstrukturierten, insbesondere von untexturierten beziehungsweise fehlerhaft texturierten Garnabschnitten in texturierten Filamentgarnen. Erfindungsgemäß kann auch vorgesehen sein, daß die Vorrichtung 15 stromabwärts, das heißt hinter dem Abzugslieferwerk 17 und vor der Garnspule 19 angeordnet ist.

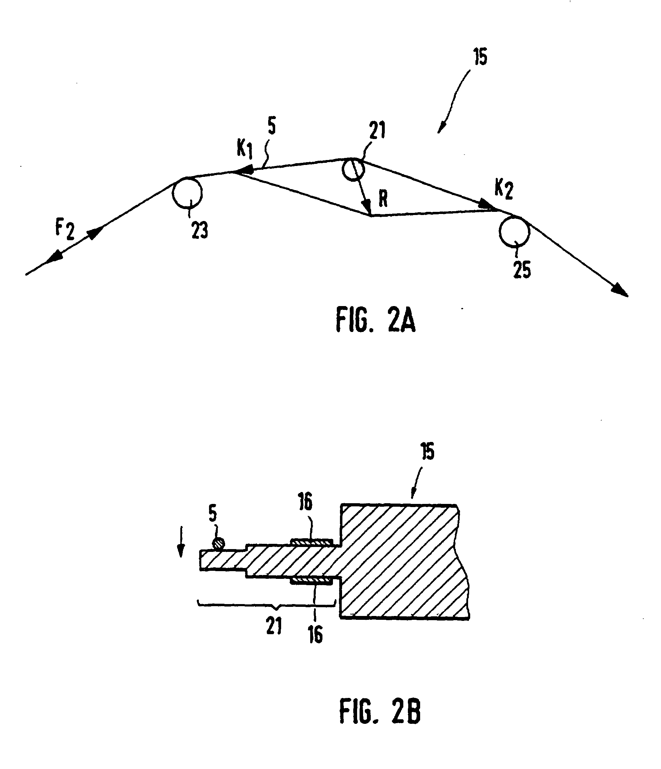

Die Figuren 2A und 2B stellen den prinzipiellen Aufbau der Kontrollvorrichtung 15 und die auf das Garn 5 einwirkenden Kraftkomponenten K1 und K2 dar. Die Vorrichtung 15 aus Meßstift 21, Dehnungsmeßstreifen 16 und einer hier nicht dargestellten Meß- und Auswerteeinrichtung mißt die Fadenzugkraft F2 hinter dem Drallgeber 13 des über den Meßstift 21 und zwei Führungsstifte 23,25 geführten Garns 5. Die Durchbiegung des Meßstifts 21 kann mit Hilfe von Dehnungsmeßstreifen 16, Hall-Sensoren, kapazitiven, induktiven oder optischen Sensoren gemessen werden. Der Figur 2A ist auch zu entnehmen, daß das Garn 5 über die Führungsstifte 23,25 und den Dehnungsmeßstift 21 läuft, so daß diese innerhalb des Krümmungsradius des Garnverlaufs angeordnet sind. In Figur 2B ist die Biegerichtung des Meßstifts 21 (Biegebalken) durch einen Pfeil angezeigt. Die Klammer zeigt den biegefähigen Bereich 21 der Vorrichtung 15 an, wobei die Dehnungsmeßstreifen 16 im Bereich der potentiell größten Durchbiegung angeordnet sind. Selbstverständlich können auch andere Meßsysteme zur Bestimmung der Fadenzugkraft verwendet werden, solange sie in der Lage sind, hochfrequente Fadenzugkraftsignale im Bereich von mindestens 0,2 kHz bis 6 kHz aufzunehmen.

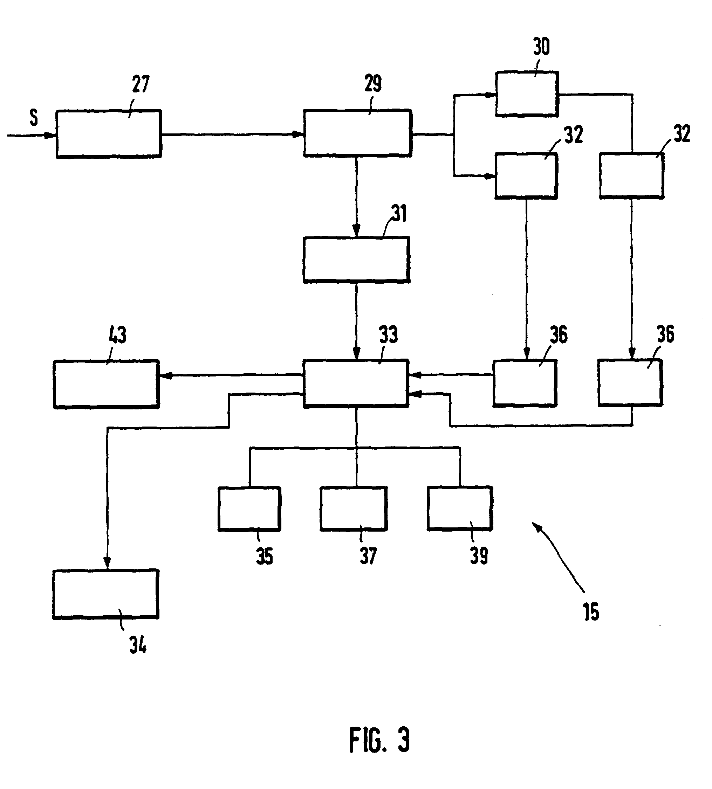

Die Figur 3 zeigt den Aufbau der Vorrichtung 15 in Form eines Blockdiagramms. Die Vorrichtung 15 weist einen Meßsensor 27 auf, dem gegebenenfalls ein hier nicht dargestellter Anpassungsverstärker zugeordnet sein kann. Die von dem Meßsensor 27 aufgenommenen und gegebenenfalls verstärkten Signale S werden durch einen Filter 29, vorzugsweise einen Tiefpaß-Filter, einem Analog/Digitalwandler 31 zugeführt. Die digitalisierten Signale werden in einem Speicher 33 gespeichert und können mittels geeigneter Vorrichtungen und Verfahren ausgewertet werden, beispielsweise indem Mittelwerte und Standardabweichungen bestimmt oder Histogramme (Feld 35) erstellt werden, oder indem eine Autokorellation (Feld 37) oder eine FFT-Analyse (Fast Fourier-Transformation) (Feld 39) durchgeführt wird. Die durch den Analog-Digitalwandler 31 digitalisierten Daten können auch einer Digitalanzeige 34 oder einem Digital/Analogwandler 43 zugeführt werden. Zur Verdichtung der Datenmengen ist es vorteilhaft, bereits das analoge Meßsignal (S) beziehungsweise das gefilterte Signal elektronisch analog aufzubereiten, beispielsweise durch analoge Differentiation, Mittelwertbildung, Bildung der Streuungsquadrate (Feld 30) und/oder Setzen von wählbaren Schwellen zur Zählung der Schwellenwertüberschreitungen (Feld 32). Die digitalen Zählergebnisse (Feld 36) stellen bereits eine extreme Verdichtung der Datenmenge dar und können an einem zentralen Auswertecomputer zur Speicherung (Feld 33) und weiteren Verarbeitung sowie zum Ausdruck beziehungsweise zur Anzeige (Feld 34) übergeben werden.

Die genannten Signalverarbeitungsvorrichtungen können Vorrichtungen zur wählbaren Vorgabe von Schwellenwerten und Zählvorrichtungen umfassen, die die Anzahlen der pro Zeiteinheit den Schwellenwert überschreitenden hochfrequenten Fadenzugkraftsignale ermitteln und zu Maße für den Grad der Garnkräuselung und für Unregelmäßigkeiten und Störstellen bereitstellen.

Die Funktion der dargestellten Vorrichtung stellt sich wie folgt dar. Das sich auf der Spule 3 befindliche, glatte Multifilamentgarn 5 wird durch das Lieferwerk 7, durch den Heizer 9, die Kühlschiene 11 und den Drallgeber 13 geführt. Der Drallgeber 13, der beispielsweise als Scheibenfrikationsaggregat ausgeführt sein kann, verdreht das Filamentgarn, wobei sich die Drehungen stromaufwärts, das heißt in Richtung Lieferwerk 7, zurückstauen. Die verdrehten Filamentgarne befinden sich in einem Spannungszustand, der durch Erhitzung im Heizer 9, beispielsweise auf 200°C, und Abkühlung auf der Kühlschiene 11 thermisch fixiert wird. Sobald das Garn 5 den Drallgeber 13 verläßt, wird die Drehung aus dem Garn 5 wieder herausgenommen, wobei der durch die thermische Fixierung erreichte Spannungszustand des Garns 5 zumindest teilweise erhalten bleibt und dazu führt, daß aus dem Drallgeber 13 ein gekräuseltes, strukturiertes, voluminöses und bauschiges Garn austritt. Dieses wird durch die Vorrichtung 15 und ein Abzugslieferwerk 17 geführt, bevor es nach Verlassen des Abzugslieferwerks 17 in entspanntem Zustand auf die Garnspule 19 aufgewickelt werden kann.

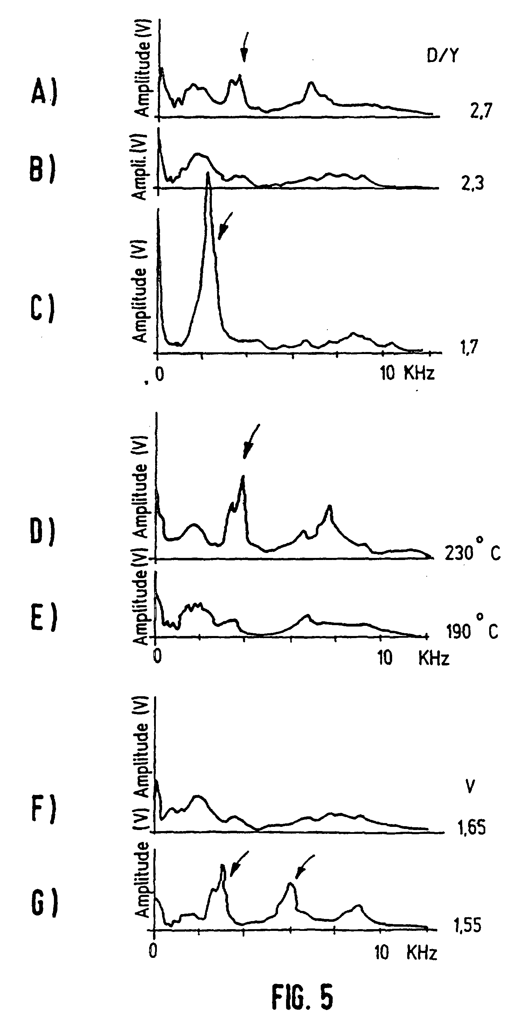

Das erfindungsgemäße Verfahren sieht nun vor, insbesondere kurze Störstellen im Garn 5, also kurze untexturierte Garnabschnitte aufzufinden, um so eine Aussage über den Grad der Garnkräuselung und deren Gleichmäßigkeit zu erhalten. Derartige kurze Störstellen im Garn 5 machen sich durch kurzzeitige Fadenzugkraftsignale bemerkbar. Das erfindungsgemäße Verfahren erlaubt eine Bestimmung und Auswertung der Garnkräuselung, in dem hochfrequente, das heißt kurzzeitig auftretende Fadenzugkraftsignale nachgewiesen werden. Dazu wird das aufgedrehte Garn 5 über den in Figur 2 gezeigten Führungsstift 23 auf einen Meßstift 21 geführt, der beispielsweise einen beziehungsweise mehrere, beispielsweise zwei oder vier, Dehnungsmeßstreifen aufweist. Das Garn 5 wird dann über einen weiteren Führungsstift 25 dem Abzugslieferwerk 17 zugeführt. Der als Meßstift aufgeführte Meßsensor 27 besitzt beispielsweise eine Eigenresonanz von bevorzugt mehr als 6 kHz, besonders bevorzugt mehr als 10 kHz, so daß hochfrequente Fadenzugkraftsignale in Frequenzbereichen von 0,2 bis 6 kHz beziehungsweise 10 kHz aufgenommen werden können. Solche Fadenzugkraftsignale können beispielsweise mit einer Abtastrate von 250 kHz gemessen und analysiert werden (Figur 5).

Die Figur 4A zeigt einen zeitlichen Fadenzugkraftverlauf F2 (Vergleich Figur 2) eines aus dem Drallgeber 13 austretenden Garns 5. Die dargestellten hochfrequenten Fadenzugkraftsignale 45 zeigen kurze Störstellen im Garn 5 an.

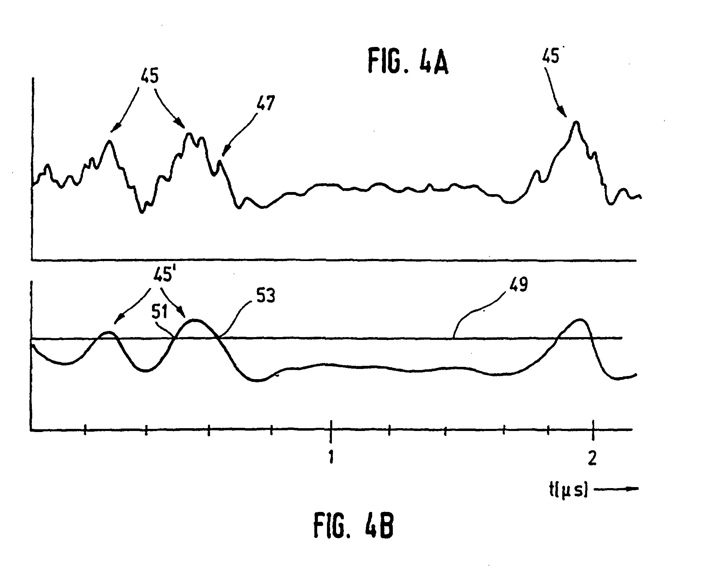

Die aufgenommenen hochfrequenten Fadenzugkraftsignale 45 werden mittels eines Tiefpaßfilters von beispielsweise etwa 60 % der Eigenresonanz geglättet, so daß die extrem hochfrequenten Fadenzugkraftsignale 47, beispielsweise über 10 kHz, ausgefiltert werden (Figur 4B), die von Vibrationen des Garns 5 auf den Friktionsscheiben herrühren. Die Figur 4B verdeutlicht ferner, daß bei der Auswertung der erhaltenen Signale ein Schwellenwert 49 vorgegeben wird, wobei eine Zählvorrichtung die Anzahl der pro Zeiteinheit den Schwellenwert 49 überschreitenden hochfrequenten Fadenzugkraftsignale 45' (Schwellenwertübertritte 51,53) registriert. Die Anzahl der pro Zeiteinheit den Schwellenwert 49 überschreitenden Fadenzugkraftsignale gibt Aufschluß über die Existenz von kurzen Störstellen im Garn 5.

Selbstverständlich ist es auch möglich, mit Hilfe des erfindungsgemäßen Verfahrens und der Vorrichtung zu dessen Durchführung längere Störstellen ("Surging"-Stellen) auf dem Garn 5 nachzuweisen.

Die Figuren 5A bis 5C zeigen Amplituden-Spektren der Fadenzugkraft F2 bei unterschiedlichen D/Y-Verhältnissen (D: Umfangsgeschwindigkeit Disk, Y: Geschwindigkeit Yarn). Bei D/Y-Verhältnissen von 1,9 bis 2,5 (Figur 5B) liegt ein stabiler, störstellenfreier Prozeß vor, während in Bereichen von D/Y < 1,9 und > 2,5 "tight spots" auftreten (Figur 5A und 5C). Für kurze Störstellen charakteristische große Aplituden sind mit einem Pfeil markiert.

Die Figuren 5D und 5E zeigen am Beispiel eines Mikrofilamentgarns PES 50 dtex f80 Amplituden-Spektren der Fadenzugkraft F2 bei unterschiedlichen Heizertemperaturen. Bei einer Heizertemperatur von 230° treten Amplituden der Fadenzugkraftschwankungen, insbesondere im hochfrequenten Bereich, auf (Figur 5D), was zu "tight spots" und Filamentbrüchen führt. Hohe Heizertemperaturen verursachen einen instabilen Prozeß, der an hochfrequenten Fadenzugkraftschwankungen erkennbar ist, und führen so zu Garnschädigungen. Figur 5E verdeutlicht das Amplituden-Spektrum eines stabilen Prozesses bei einer Heizertemperatur von 190°C.

Die Figuren 5F und 5G zeigen Amplituden-Spektren der Fadenzugkraft F2 bei unterschiedlichen Verstreckungsgraden V. Bei niedriger Verstreckung (V = 1,55) kommt es zur Bildung von Störstellen, bei denen Verstreckungsgrad von V = 1,60 liegt ein stabiler Prozeß liegen vor.

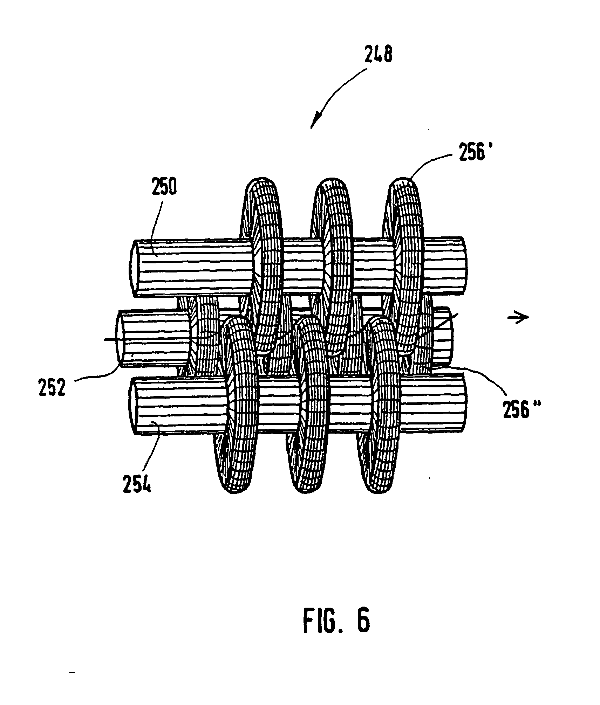

Die Figur 6 zeigt schematisch den Aufbau eines als Friktionsaggregat 248 ausgebildeten Drallgebers 13. Die Figur 6 verdeutlicht den Aufbau des drei Achsen 250, 252, 254 aufweisenden Scheibenfriktionsaggregates 248. Auf jeder der drei Achsen 250, 252, 254 sind konzentrisch Friktionsscheiben 256 angeordnet. Die Garnlaufrichtung VF ist durch einen Pfeil dargestellt. Die erfindungsgemäß vorgesehene Bestimmung der Garndicke beziehungsweise des Garnlaufverhaltens wird bevorzugt an oder auf der letzten Arbeitsscheibe 256' oder der Ausgangsscheibe 256" durchgeführt.

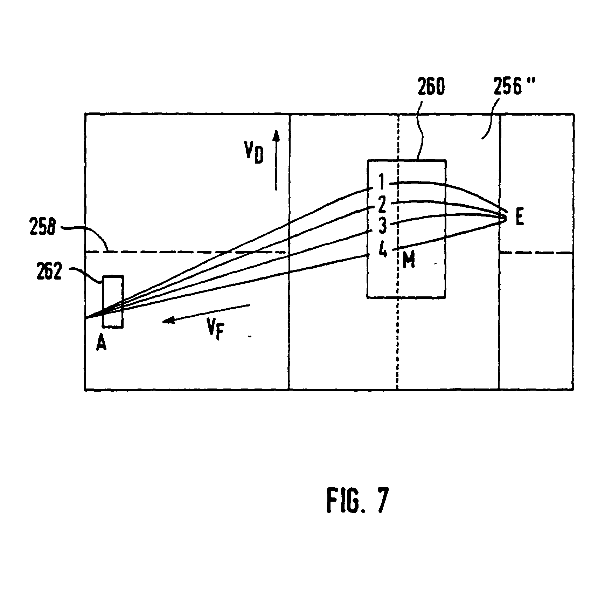

Die Figur 7 stellt schematisch die Garnlaufgeometrie auf der Ausgangsscheibe 256" bei unterschiedlichen Prozeßbedingungen dar. Die Figur verdeutlicht den Verlauf des Garns in Richtung VF vom Einlaufpunkt E über die Ausgangsscheibe 256" mit deren Äquator M (Scheibenmitte) in Richtung Auslaufpunkt A des Aggregates 248. Dargestellt ist ferner die Scheibendrehachse 258. Das Garnlaufverhalten und/oder die Garndicke auf der Ausgangsscheibe 256" wurde mittels einer High-speed-Videokamera mit Auswertesystem bestimmt, die bis maximal 6000 Bilder/s aufnehmen kann. Die optische oder laseroptische Bestimmung der Garndicke und/oder des Garnlaufverhaltens läßt sich auf (beispielsweise Feld 260) oder hinter der Ausgangsscheibe 256" (beispielsweise Feld 262) durchführen. Den Aufnahmen der High-speed-Videokamera läßt sich entnehmen, daß sich der Faden grundsätzlich auf der Scheibe 256" quer zur Fadenlaufrichtung VF, das heißt in der Richtung VD bewegt, und zwar sowohl bei einem stabilen Prozeß als auch bei einem instabilen, das heißt zu Störstellen führendem Texturierungsprozeß. Bei einem stabilen Prozeß, das heißt einem Prozeß, bei dem keine Störstellen auftreten, verläuft der Faden auf einer stark gekrümmten Kurve (Position 1). Die Amplitude der Garnquerbewegung ist klein. Die maximale Amplitude der Garnquerbewegung in der Scheibenmitte M liegt bei ca. 0,2 mm (Durchmesser des verwendeten PES-Garns 50 dtext f 80 im Friktionsaggregat liegt bei 0,09 mm, Garnlaufgeschwindigkeit beispielsweise 600 m/min).

Bei einem leicht instabilen Prozeß tritt eine relativ große Garnquerbewegung auf, wobei die maximale Amplitude der Garnquerbewegung bei 0,5 mm liegt. Der Faden schwingt periodisch mit niedriger Frequenz zwischen den Positionen 1 und 3 hin und her.

Bei einem ausgesprochen instabilen Prozeß befindet sich der Faden meistens in der Position 4. Der Faden schwingt periodisch mit niedriger Frequenz zwischen den Positionen 4 und 2 hin und her, wobei die maximale Amplitude der Garnquerbewegungen bei ca. 1 mm liegt.

Unerwünschte kurze, untexturierte Stellen des Garns ("tight spots") treten auf, wenn der Prozeß instabil ist.

Da der Nachweis von Störstellen in texturierten Garnen auch mittels Messung der Fadenzugkraft im kHz-Frequenzbereich möglich ist, können die erfindungsgemäßen optischen Verfahren dazu verwendet werden, Fadenzugkraftmessungen zu überprüfen oder zu ersetzen. Erfindungsgemäß können Ergebnisse der Garnlaufverhalten oder Garndickenbestimmung mit Ergebnissen der Fadenzugkraftmessung korreliert werden, um "tight spots" nachzuweisen. Der Faden neigt bei höheren Fadenzugkräften zu einem geraden, schwächer gekrümmten Lauf über die Scheibe 256", das heißt er versucht, den kürzesten Weg einzuschlagen. Dabei kann der Faden beispielsweise von Position 1 auf Position 2 rutschen, wobei Drallschlupf und unerwünschte "tight spots" entstehen können. Die Garndicke ändert sich bei einem stabilen Prozeß in der M-A-Zone (vgl. Figur 7) kaum, während ein instabiler Prozeß unter Bildung kurzer Störstellen durch schwankende Garndicke erkennbar wird.

Selbstverständlich ist es mit den erfindungsgemäßen Verfahren nicht nur möglich, kurze Störstellen, sondern auch lange, untexturierte Gamabschnitte ("Surging-Stellen") nachzuweisen.

## Patentansprüche

1. Verfahren zum Nachweis von untexturierten beziehungsweise fehlerhaft texturierten Garnabschnitten in texturierten Filamentgarnen während des Texturierprozesses, wobei das texturierte Garn laufend gemessen wird, **dadurch gekennzeichnet, daß** hochfrequente Meßsignale in einem Frequenzbereich von 0,2 bis 10 kHz erzeugt und gemessen werden, und als Meßsignale Fadenzugkraftsignale mittels eines das Garn abtastenden Meßsystems als Nachweise für untexturierte beziehungsweise fehlerhaft texturierte Garnabschnitte bestimmt, aufbereitet und auf den Grad der Garnkräuselung und deren Gleichmäßigkeit hin ausgewertet werden.

2. Verfahren nach Anspruch 1, **dadurch gekennzeichnet, daß** die Meßsignale in einem Frequenzbereich von von 1 bis 6 kHz erzeugt und gemessen werden.

3. Verfahren nach Anspruch 1 und 2, **dadurch gekennzeichnet, daß** die gemessenen Fadenzugkraftsignale vor der Auswertung aufbereitet, zum Beispiel gefiltert werden.

4. Verfahren nach einem der Ansprüche 2 oder 3, **dadurch gekennzeichnet, daß** Fadenzugkraftsignale mit Frequenzen über 6 kHz ausgefiltert werden.

5. Verfahren nach einem der vorangehenden Ansprüche, **dadurch gekennzeichnet, daß** der Texturierprozeß ein Friktionsfalschdrahtverfahren ist.

6. Verfahren nach einem der vorangehenden Ansprüche, **dadurch gekennzeichnet, daß** die Fadenzugkraftsignale gemessen werden, nachdem das Filamentgarn einen Drallgeber durchlaufen hat.

7. Verfahren nach einem der vorangehenden Ansprüche, **dadurch gekennzeichnet, daß** das Filamentgarn aus einem Thermoplast, wie Polyester, Polyethylen, Polyamid oder Polypropylen, besteht.

8. Verfahren nach einem der vorangehenden Ansprüche, **dadurch gekennzeichnet, daß** die gemessenen, aufbereiteten und gefilterten Signale mittels Bestimmung des Mittelwerts, der Standardabweichung, der Schwellenwertüberschreitungen, der Erstellung eines Amplitudenhistogramms, eines Amplitudenspektrums, der FFT-Analyse (Fast Fourier Transformation-Analyse) oder der Autokorrelation ausgewertet werden.

9. Verfahren zum Nachweis von untexturierten beziehungsweise fehlerhaft texturierten Garnabschnitten in texturierten Filamentgarnen während des Texturierprozesses, wobei das texturierte Garn laufend gemessen wird, insbesondere nach einem der vorangehenden Ansprüche, **dadurch gekennzeichnet, daß** hochfrequente Meßsignale in einem Frequenzbereich von 0,2 bis 10 kHz erzeugt und gemessen werden, und als Meßsignale mittels das Garn optisch oder laseroptisch abtastender Vorrichtungen optische und/oder laseroptische Meßsignale über die Garndicke beziehungsweise Abweichungen von der mittleren Garndicke bestimmt und anschließend ausgewertet werden.

10. Verfahren zum Nachweis von untexturierten beziehungsweise fehlerhaft texturierten Garnabschnitten in texturierten Filamentgarnen während des Texturierprozesses, wobei das texturierte Garn laufend gemessen wird, insbesondere nach einem der vorangehenden Ansprüche, **dadurch gekennzeichnet, daß** hochfrequente Meßsignale in einem Frequenzbereich von 0,2 bis 10 kHz erzeugt und gemessen werden, und als Meßsignale mittels das Garn optisch oder laseroptisch abtastender Vorrichtungen optische und/oder laseroptische Meßsignale über die Garnposition und/oder das Garnlaufverhalten, insbesondere die Garnquerbewegung, bestimmt und ausgewertet wird.

11. Verfahren nach einem der Ansprüche 9 oder 10, **dadurch gekennzeichnet, daß** kurze, untexturierte Garnabschnitte nachgewiesen werden.

12. Verfahren nach einem der Ansprüche 9 bis 11, **dadurch gekennzeichnet, daß** die optische und/oder laseroptische Bestimmung mittels Vorrichtungen durchgeführt wird, die hohe Abtastraten >10 kHz, aufweisen.

13. Verfahren nach einem der Ansprüche 9 bis 12, **dadurch gekennzeichnet, daß** die optische und/oder laseroptische Bestimmung während der im Texturierprozeß erfolgenden Drallerteilung im Bereich der Friktionsscheiben eines Scheibenfriktionsaggregates durchgeführt wird.

14. Verfahren nach einem der Ansprüche 9 bis 13, **dadurch gekennzeichnet, daß** die optische und/oder laseroptische Bestimmung an oder auf der letzten Arbeitsscheibe oder der Ausgangsscheibe eines Scheibenfriktionsaggregates durchgeführt wird.

15. Verfahren nach einem der Ansprüche 9 bis 14, **dadurch gekennzeichnet, daß** die optische und/oder laseroptische Bestimmung auf der Strecke zwischen Drallgeber und Aufwindegarnspule durchgeführt wird.

16. Verfahren nach einem der Ansprüche 10 bis 16, **dadurch gekennzeichnet, daß** die bestimmten optischen und/oder laseroptischen Daten mit Ergebnissen von Fadenzugkraftmessungen korreliert und ausgewertet werden.

17. Vorrichtung zum Nachweis von untexturierten beziehungsweise fehlerhaft texturierten Garnabschnitten in texturierten Filamentgarnen, insbesondere zur Durchführung eines Verfahrens nach einem der Ansprüche 1 bis 8, welches einen Meßsignalsensor, eine Vorrichtung zur Signalaufbereitung und Signalverarbeitung sowie Vorrichtungen zur Signalanalyse und Datenverarbeitung umfaßt, **dadurch gekennzeichnet, daß** der Meßsensor (27) dazu ausgebildet ist, Fadenzugkraftsignale (45) mit hohen Frequenzen in einem Bereich von 0,2 bis 10 kHz aufzunehmen und daß eine einen Schwellenwert (49) wählbar vorgebbare Schwellenwertvorrichtung und eine Zählvorrichtung vorgesehen sind, wobei letztere die Anzahl der hochfrequenten Fadenzugkraftsignale (45) erfaßt, die in einer bestimmten Zeit den Schwellenwert (49) überschreiten.

18. Vorrichtung nach Anspruch 17, **dadurch gekennzeichnet, daß** der Meßsensor (27) einen biegefähigen Meßstift (21) aufweist, worüber das Garn führbar ist, und die Durchbiegung des Meßstifts (21) mittels Dehnungsmeßstreifen (16), Hall-Sensoren, kapazitiven, induktiven oder optischen Sensorelementen gemessen werden.

## Claims

1. Method for detecting untextured or incorrectly textured yarn sections in textured filament yarns during the texturing process, wherein the textured yarn is continuously measured, **characterised in that** high-frequency measuring signals in a frequency range from 0.2 to 10 kHz are generated and measured, and yarn tensile force signals are determined as measuring signals by means of a measuring system, which scans the yarn, as verification of untextured or incorrectly textured yarn sections, conditioned and evaluated as to the degree of yarn crimping and the uniformity thereof.

2. Method according to Claim 1, **characterised in that** the measuring signals are generated in a frequency range of from 1 to 6 kHz and measured.

3. Method according to Claims 1 and 2, **characterised in that** the measured yarn tensile force signals are conditioned before being evaluated, for example filtered.

4. Method according to any one of Claims 2 and 3, **characterised in that** yarn tensile force signals of frequencies exceeding 6 kHz are filtered out.

5. Method according to any one of the preceding Claims, **characterised in that** the texturing process is a false friction twist method.

6. Method according to any one of the preceding Claims, **characterised in that** the yarn tensile force signals are measured after the filament yarn has passed through a twisting element.

7. Method according to any one of the preceding Claims, **characterised in that** the filament yarn consists of a thermoplastic such as polyester, polyethylene, polyamide or polypropylene.

8. Method according to any one of the preceding Claims, **characterised in that** the measured, conditioned and filtered signals are evaluated by determining the mean value, the standard deviation, the threshold value exceedance occurrences, the creation of an amplitude histogram, an amplitude spectrum, the FFT analysis (fast Fourier transform analysis) or autocorrelation.

9. Method for detecting untextured or incorrectly textured yarn sections in textured filament yarns during the texturing process, wherein the textured yarn is continuously measured, in particular according to any one of the preceding Claims, **characterised in that** high-frequency measuring signals in a frequency range from 0.2 to 10 kHz are generated and measured, and optical and/or laser-optical measuring signals relating to the yarn thickness or deviations from the mean yarn thickness are determined as measuring signals by means of devices which scan the yarn in optical or laser-optical fashion, and then evaluated.

10. Method for detecting untextured or incorrectly textured yarn sections in textured filament yarns during the texturing process, wherein the textured yarn is continuously measured, in particular according to any one of the preceding Claims, **characterised in that** high-frequency measuring signals in a frequency range from 0.2 to 10 kHz are generated and measured, and optical and/or laser-optical measuring signals relating to the yarn position and/or the yarn running behaviour, in particular the transverse yarn movement, are determined as measuring signals by means of devices which scan the yarn in optical or laser-optical fashion, and evaluated.

11. Method according to any one of Claims 9 and 10, **characterised in that** short, untextured yarn sections are detected.

12. Method according to any one of Claims 9 to 11, **characterised in that** the optical and/or laser-optical determination is carried out by means of devices having high scanning rates > 10 kHz.

13. Method according to any one of Claims 9 to 12, **characterised in that** the optical and/or laser-optical determination is carried out during the twisting taking place in the texturing process in the region of the friction discs of a disc friction unit.

14. Method according to any one of Claims 9 to 13, **characterised in that** the optical and/or laser-optical determination is carried out at or on the last working disc or the initial disc of a disc friction unit.

15. Method according to any one of Claims 9 to 14, **characterised in that** the optical and/or laser-optical determination is carried out along the stretch between the twisting element and the yarn take-up bobbin.

16. Method according to any one of Claims 10 to 16, **characterised in that** the optical and/or laser-optical data which are determined are correlated with results of yarn tensile force measurements and evaluated.

17. Device for detecting untextured or incorrectly textured yarn sections in textured filament yarns, in particular for carrying out a method according to any one of Claims 1 to 8, which comprises a measuring signal sensor, a device for signal conditioning and signal processing as well as devices for signal analysis and data processing, **characterised in that** the measuring sensor (27) is formed to pick up yarn tensile force signals (45) of high frequencies in a range from 0.2 to 10 kHz, and that a threshold value device, which selectively predetermines a threshold value (49), and a counting device are provided, wherein the latter records the number of high-frequency yarn tensile force signals (45) which exceed the threshold value (49) in a certain period.

18. Device according to Claim 17, **characterised in that** the measuring sensor (27) comprises a flexible measuring pin (21), over which the yarn can be guided, and the deflection of the measuring pin (21) is measured by means of strain gauges (16), Hall sensors, capacitive, inductive or optical sensor elements.

## Revendications

1. Procédé de détection, pendant l'opération de texturation, de tronçons de fil non texturés ou incorrectement texturés de fils à filaments texturés, le fil texturé étant mesuré en permanence, **caractérisé en ce que** des signaux de mesure à haute fréquence, dans une plage de fréquences de 0,2 à 10 kHz, sont créés et mesurés, et pour la détection des tronçons de fil non texturés ou incorrectement texturés, on utilise comme signaux de mesure des signaux de force de traction sur le fil qui sont définis au moyen d'un système de mesure balayant le fil et transformés, et le degré de frisage du fil et sa régularité sont évalués.

2. Procédé selon la revendication 1, **caractérisé en ce que** les signaux de mesure sont créés et mesurés dans une plage de fréquences de 1 à 6 kHz.

3. Procédé selon les revendications 1 et 2, **caractérisé en ce que** les signaux de force de traction mesurés sur le fil sont transformés, par exemple filtrés, avant leur évaluation.

4. Procédé selon l'une des revendications 2 ou 3, **caractérisé en ce que** les signaux de force de traction sur le fil sont filtrés à des fréquences supérieures à 6 kHz.

5. Procédé selon l'une des revendications précédentes, **caractérisé en ce que** l'opération de texturation est un procédé de friction à fausse torsion.

6. Procédé selon l'une des revendications précédentes, **caractérisé en ce que** les signaux de force de traction sur le fil sont mesurés après que le fil de filaments ait traversé un dispositif de torsion.

7. Procédé selon l'une des revendications précédentes, **caractérisé en ce que** le fil de filaments est réalisé en un thermoplastique, par exemple le polyester, le polyéthylène, le polyamide ou le polypropylène.

8. Procédé selon l'une des revendications précédentes, **caractérisé en ce que** les signaux mesurés, traités et filtrés sont évalués par détermination de la valeur moyenne, de l'écart-type, des dépassements de valeur de seuil, établissement d'un histogramme d'amplitude et d'un spectre d'amplitude, analyse par transformée rapide de Fourier et autocorrélation.

9. Procédé de détection, pendant l'opération de texturation, de tronçons de fil non texturés ou incorrectement texturés dans des fils de filaments texturés, dans lequel le fil texturé est mesuré en continu, en particulier selon l'une des revendications précédentes, **caractérisé en ce que** des signaux de mesure à haute fréquence, dans une plage de fréquences de 0,2 à 10 kHz, sont créés et mesurés, et comme signaux de mesure, des signaux de mesure optiques et/ou optiques par laser concernant l'épaisseur du fil ou des écarts par rapport à l'épaisseur moyenne du fil sont définis au moyen de dispositifs balayant le fil par voie optique ou optique par laser et sont ensuite évalués.

10. Procédé de détection, pendant l'opération de texturation, de tronçons de fil non texturés ou incorrectement texturés dans des fils de filaments texturés, dans lequel le fil texturé est mesuré en permanence, en particulier selon l'une des revendications précédentes, **caractérisé en ce que** des signaux de mesure à haute fréquence, dans une plage de fréquences de 0,2 à 10 kHz, sont créés et mesurés, et comme signaux de mesure, des signaux de mesure optiques et/ou optiques par laser concernant la composition du fil et/ou le déplacement du fil, en particulier le déplacement du fil dans le sens transversal, sont définis au moyen de dispositifs balayant le fil par voie optique ou optique par laser et sont ensuite évalués.

11. Procédé selon l'une revendication 9 ou 10, **caractérisé en ce que** l'on détecte de courts tronçons de fil non texturés.

12. Procédé selon l'une des revendications 9 à 11, **caractérisé en ce que** la détermination optique et/ou optique par laser est réalisée au moyen de dispositifs qui présentent des taux de balayage élevés, supérieurs à 10 kHz.

13. Procédé selon l'une revendications 9 à 12, **caractérisé en ce que** la détermination optique et/ou optique par laser est réalisée pendant la transmission d'une torsion, qui a lieu au cours de l'opération de texturation dans la région de la plaque de frottement d'une garniture à plaque de frottement.

14. Procédé selon l'une des revendications 9 à 13, **caractérisé en ce que** la détermination optique et/ou optique par laser est réalisée contre ou sur la dernière plaque de travail ou sur la plaque de sortie d'une garniture à plaque de frottement.

15. Procédé selon l'une des revendications 9 à 14, **caractérisé en ce que** la détermination optique et/ou optique par laser est réalisée sur le parcours situé entre le dispositif de torsion et la bobine de bobinage de fil.

16. Procédé selon l'une revendications 10 à 16, **caractérisé en ce que** les données optiques et/ou optiques par laser déterminées sont corrélées aux résultats de mesure de la force de traction sur le fil et évaluées.

17. Dispositif de détection de tronçons de fil non texturés ou incorrectement texturés dans des fils de filaments texturés, en particulier en vue de la mise en oeuvre d'un procédé selon l'une des revendications 1 à 8, qui comprend un capteur de signaux de mesure, un dispositif de préparation du signal et de transformation du signal ainsi que des dispositifs d'analyse du signal et de traitement des données, **caractérisé en ce que** le capteur de mesure (27) est configuré pour des signaux (45) de force de traction sur le fil à haute fréquence, dans une plage de 0,2 à 10 kHz, et en ce qu'un dispositif à valeur de seuil qui est apte à prédéterminer de façon sélectionnable une valeur de seuil (49), ainsi qu'un dispositif de comptage sont prévus, ce dernier déterminant le nombre des signaux (45) à haute fréquence de force de traction sur le fil, qui dépassent la valeur de seuil (49) pendant une durée donnée.

18. Dispositif selon la revendication 17, **caractérisé en ce que** le capteur de mesure (27) présente une tige de mesure (21) apte à se fléchir, sur laquelle le fil peut être guidé, et la flexion de la tige de mesure (21) est mesurée au moyen de rubans (16) de mesure d'allongement, de capteurs Hall ou d'éléments de capteur capacitifs, inductifs ou optiques.
